# EUROPEAN PATENT SPECIFICATION

(11) **EP 3 710 071 B1**
(45) Date of publication and mention of the grant of the patent: **10.12.2025**
(21) Application number: 18833287.8
(22) Date of filing: 13.11.2018
(51) Int. Cl.: A61L 9/012, A61L 9/14, B82Y 30/00, D06M 13/00

(54) **FRESHENER FOR SPACES AND OBJECTS**
AUFFRISCHER FÜR RÄUME UND GEGENSTÄNDE
DÉSODORISANT D'ESPACES ET D'OBJETS

(30) Priority: 17.11.2017 HR 20171785
(43) Date of publication of application: 23.09.2020
(73) Proprietor: Oxylus d.o.o., 31000 Osijek (HR)
(72) Inventor: DOMAZET, Ivo, 31000 Osijek (HR)
(74) Representative: Strniscak, Tomislav
(86) International application number: PCT/HR2018/000020
(87) International publication number: WO 2019/097266

(56) References cited:
- EP-A2- 1 495 102
- EP-B1- 1 495 102
- US-A1- 2007 037 732

## Description

### FIELD OF THE INVENTION

The present invention relates to fresheners for spaces and objects and substances which release fragrances, and the application procedure thereof.

### TECHNICAL PROBLEM

Usually, the objects that release fragrances are manufactured in such a way that the fragrances are applied onto these objects; afterward, such products are packed in plastic packaging that does not release fragrances, which plastic packaging is removed from these objects during use. Such objects have i.e. release intensive fragrance at the beginning, and, as time goes by, the fragrance intensity is weakened and finally vanishes. The problem lies in the fact that, once the object is taken out of the plastic packaging, it is no longer possible to control neither intensity nor quantity of the releasing fragrance. Hence, the object releases fragrance, whereby the fragrance is spent uncontrollably.

The existing solutions in space freshening are mainly based on devices that occasionally inject a certain fragrant substances into the space, or the fragrant substances are permanently released into the space. However, with these methods there is no control, whether the people for whom the fragrant substance is released into the space, actually are in the space.

With the present invention, the inventor has solved the problem how to save on consumption of fragrant substances in order to be consumed only when persons are present in the space, namely that the object intended to be freshened with fragrance releases fragrance only when in use, i.e. when people use it.

### PRIOR ART

There are products on the market onto which, by special procedures, the fresheners for objects are applied, which fresheners need to be scratched on the surface to release the fragrance; however since the fragrance carrier or the medium which releases the fragrance is not in the capsule, the scent particles are still slowly leaving in the atmosphere and after a while, such space freshener no longer has its function. The technological procedures used for applying such fresheners for objects are not suitable for personal use.

There are also space fresheners that spray fragrance (perfume) into the space in certain time sequences. However, the problem is that such fragrances are being sprayed irrespectively of the presence of persons. Such fragrances, once they get into the space, quickly lose their scent intensity and need to be added again.

Also, perfumes and cologne waters that are applied to the skin or clothing by spraying or otherwise commonly loose a scent within 24 hours. According to the researches carried out by the inventor, the capsules revealed in the present invention retain the fragrance irrespectively of whether they were exposed to high temperature up to +40 ° C or low temperature to -20 ° C even several months after being exposed to such weather conditions.

Relevant prior art includes US 2007/0037732 A1, relating to fragrance compositions, and EP 1 495 102 B1, concerning compositions comprising a dispersant and microcapsules containing an active material.

The document US 2007/0037732 A1 discloses a freshener containing a fragrance composition but does not disclose that the fragrance composition comprises demineralised water, a viscosity modifier, sodium chloride, and a preservative.

The document EP 1 495 102 B1 similarly discloses a freshener with microencapsulated active materials but fails to disclose that the fragrance composition includes sodium chloride.

Accordingly, the inventor considers that neither of these documents discloses, nor renders obvious, the invention as described and claimed in the present application.

### BRIEF SUMMARY OF THE PRESENT INVENTION:

The freshener revealed here is used for perfuming and freshening various objects and spaces. It comprises several components, one of which is in the form of a capsule which contains a fragrance. The capsule may be a microcapsule or a nanocapsule. It is applied by spraying directly on the fabric or surface. A pleasant, floral fragrance smells from the first application, however, the specificity of this freshener is noticeable after it is dried on the treated surface. After application by spraying and the time of drying, in physical contact with the treated surface the capsules break and release the fragrance into the space. This freshener is used on all types of textiles, leather, artificial leather, but also on hard and less absorbent surfaces. Mechanic force / friction (sitting, walking and the like) cause the breaking of the fragrant capsules that remain on the fibres, thereby obtaining the additional "boost" of fragrance. By its very nature, the product is completely safe to use, it does not comprise hazardous components. These capsules provide longer lasting freshness because the fragrance is protected as long as the capsules are intact. Only after application of a force, by breaking capsules, a pleasant fragrance is released.

Freshener for spaces and objects revealed therein comprises capsules which contain a fragrant composition, wherein, when a single capsule breaks, the scent of the fragrant composition is released into the space and wherein the subject fresheners for spaces and objects provide a longer lasting freshness, as the fragrance is protected in each capsule as long as the said capsule remains intact. The fragrant composition in such capsule comprises demineralised water, a viscosity modifier, sodium chloride and preservative. Usually, the viscosity modifier is a cellulose gum or an acrylic copolymer. Such capsules may either be nano-capsules or microcapsules. Methods for producing such capsules are either one of the nanoencapsulation methods, or one of the microencapsulation methods.

The essences of this invention in fact are the capsules of fragrance that break by physical contact and release a pleasant fragrance. This freshener is long-lasting and very economical, does not spend over time if it is left untouched, it does not damage any surfaces and does not leave non-washable traces, it is easy to use and apply. It can also be used in washing machines for laundry, carpets and upholstery, it is excellent and adheres well to all types of textiles, leather, artificial leather, as well as on hard and less absorbent surfaces.

Capsules may have, but not necessarily, "release on demand", "slow release" or "high diffusion" effects, or any of the aforementioned. In the case of "release on demand", after the application of force, the membrane of the fragrance capsule breaks and the fragrance is released at once. In the case of a "slow release", after the application of force, the membrane of the fragrance capsule cracks and, after releasing the initial noticeable amount of the fragrance, it continues to release fragrance over a certain period of time (even up to several months). The embodiment marked as the "High Diffusion" means that after the capsule is activated by touch, the capsule has a better / stronger instantaneous release of the fragrance, but also the effect of prolonged action / fragrance release.

The usual manner of use of this freshener for spaces and objects is to apply it by spraying directly onto the surface or the fabric. Such freshener may also be applied to objects by sprinkling onto the surface.

This freshener is especially suitable for freshening of the fabrics and textiles.

### DETAILED DESCRIPTION OF AT LEAST ONE WAY OF CARRYING OUT THE INVENTION

One of the possible methods of preparation of this freshener for spaces and objects is to prepare a fragrant blend comprising 50-100 parts by mass of demineralised water, 0-10 parts by mass of viscosity modifier, 0-10 parts by mass of sodium chloride, 0-10 parts by mass of nano-encapsulated fragrance and 0-1 parts by mass of preservative.

Afterwards with such a mixture, by nanoencapsulation method, the nano-capsules are made. The resulting mixture i.e. the nanocapsules are mixed with a particular medium and filled into pressurized or non-pressurized vials.

The method of using the subject freshener for spaces and objects is to apply it by spraying directly onto the surface or the fabric. It takes some time for the applied freshener to dry. A fragrance occurs when the nanocapsules break, usually due to pressure or friction which they are exposed to, for example, by walking on the surface comprising nanocapsules, sitting on the chair on the surface of which are the nanocapsules, wiping with the towel with nanocapsules, etc.

This freshener may also be applied by coating, sprinkling or immersion.

After the drying time and water evaporation, a thin layer of nanocapsules with fragrance is formed on the treated surface. Typical applications are in transport and carriage of passengers and goods, tourism, foodservice sector and hospitality industries, public institutions and public facilities, health facilities, sport facilities, wherein the freshener is applied onto the carpets, upholstered furniture and seats, seats and floors in vehicles for passenger transport and transporting goods, on the bed linen, napkins, tablecloths, curtains, towels, upholstery, and clothing.

A commonly used preservative is either 5-chloro-2-methyl-4-isothiazolin-3-one and 2-methyl-4-isothiazolin-3-one and benzyl alcohol or 1,2-benzisothiazol-3 (2H)-one and 3(2H)-Isothiazolone, 2-Methyl- or 1,2-benzisothiazol-3(2H)-one, and 2-methylisothiazol-3(2H)-one or 1,3-Bis (hydroxymethyl)-5,5-dimethylimi-dazolidine-2,4-dione and Formaldehyde or 1,2-benzisothiazol-3(2H)-one and 2-Methyl-2H-isothiazol-3-one and N-(3-Aminopropyl)-N-dodecylpropane-1,3 diamine.

In certain embodiments, this freshener for spaces and objects comprises capsules of such characteristics that after the application of the force onto the capsule, the membrane of the fragrance capsule breaks and the fragrance is released at once. In another embodiment, this freshener comprises capsules of such characteristics that after the application of the force onto the capsule, the membrane of the fragrance capsule cracks and, after releasing the initial noticeable amount of the fragrance, it continues to release the fragrance over a certain period of time up to several months. In further embodiment, this freshener for spaces and objects comprises capsules of such characteristics that after the activation of the capsule by touch, the capsule has a better and stronger instantaneous release of the fragrance, but also the effect of prolonged action / fragrance release.

It is possible that in one embodiment all three types of capsules are used, so that this freshener for spaces and objects comprises capsules of such characteristics that after the application of force on the capsule, the membrane of the fragrance capsule cracks and, after releasing the initial noticeable amount of the fragrance, it continues to release fragrance over a certain period of time up to several months and / or after the application of the force onto the capsule, the membrane of the fragrance capsule breaks and the fragrance is released at once and / or after the activation of the capsule by touch, the capsule has a better and stronger instantaneous release of the fragrance, but also the effect of prolonged action / fragrance release.

In one embodiment, instead of the fragrance, the capsule may comprise odour neutralizer, i.e. antiodour, whose function would be, instead of scenting the objects, to neutralize the smell, i.e. the bad odours.

## Claims

1. A freshener for spaces and objects comprising nano-capsules containing a fragrant composition,
**characterized in that** the fragrant composition is configured :
- to release the fragrance of the fragrant composition into the space when a single nano-capsule breaks, and
- to provide freshness as the fragrance is protected in each nano-capsule as long as said nano-capsule remains intact,
and wherein the fragrant composition comprises:
- demineralised water,
- a viscosity modifier,
- sodium chloride, and
- a preservative,
wherein the viscosity modifier is selected from cellulose gum or an acrylic copolymer, and wherein the nano-capsules are produced by a method of nanoencapsulation and wherein said freshener is configured to spray or sprinkle said nanocapsules on surfaces or fabrics.

2. Freshener for spaces and objects, as claimed in any of the claims from 1. to 3., **characterized in that** the preservative is 5-chloro-2-methyl-4-isothiazolin-3-one and 2-methyl-4-isothiazolin-3-one and benzyl alcohol.

3. Freshener for spaces and objects, as claimed in any of the claims from 1. to 3., **characterized in that** the preservative is 1,2-benzisothiazol-3 (2H)-one and 3(2H)-Isothiazolone, *2-Methyl-.*

4. Freshener for spaces and objects, as claimed in any of the claims from 1. to 3., **characterized in that** the preservative is 1,2-benzisothiazol-3(2H)-one and 2-methyl-(2H)-isothiazol-3-one

5. Freshener for spaces and objects, as claimed in any of the claims from 1. to 3., **characterized in that** the preservative is 1,3-Bis (hydroxymethyl)-5,5-dimethylimi-dazolidine-2,4-dione and Formaldehyde.

6. Freshener for spaces and objects, as claimed in any of the claims from 1. to 3., **characterized in that** the preservative is 1,2-benzisothiazol-3(2H)-one and 2-Methyl-2H-isothiazol-3-one and N-(3-Aminopropyl)-N-dodecylpropane-1,3-diamine.

7. Freshener for spaces and objects, as claimed in any of the previous claims, **characterized in that** the nano-capsules have such characteristics that after the application of the force onto the capsule, the membrane of the fragrance capsule breaks and the fragrance is released at once.

8. Freshener for spaces and objects, as claimed in any of the previous claims from 1. to 6. , **characterized in that** the capsules have such characteristics that after the application of the force onto the capsule, the membrane of the fragrance capsule cracks and, after releasing the initial noticeable amount of the fragrance, it continues to release the fragrance over a certain period of time up to several months.

9. Freshener for spaces and objects, as claimed in any of the previous claims from 1. to 6., **characterized in that** the capsules have such characteristics that after the capsule is activated by touch, the capsule has a better / stronger instantaneous release of the fragrance, but also the effect of prolonged action / fragrance release.

10. Freshener for spaces and objects, as claimed in any of the previous claims from 1. to 6. , **characterized in that** the capsules have such characteristics that after the application of the force onto the capsule, the membrane of the fragrance capsule cracks and, after releasing the initial noticeable amount of the fragrance, it continues to release the fragrance over a certain period of time up to several months and / or that after the application of the force onto the capsule, the membrane of the fragrance capsule breaks and the fragrance is released at once and/or that after the capsule is activated by touch, the capsule has a better / stronger instantaneous release of the fragrance, but also the effect of prolonged action / fragrance release.

11. Freshener for spaces and objects, as claimed in any of the previous claims, **characterized in that** it is used for freshening of the fabrics and textiles.

## Patentansprüche

1. Auffrischer für Räume und Gegenstände, umfassend Nanokapseln, die eine duftende Zusammensetzung enthalten,
**dadurch gekennzeichnet, dass** die duftende Zusammensetzung so ausgestaltet ist,
- dass beim Aufbrechen einer einzelnen Nanokapsel der Duft der duftenden Zusammensetzung in den Raum freigesetzt wird, und
- dass eine Frischewirkung bewirkt wird, da der Duft in jeder Nanokapsel geschützt ist, solange die betreffende Nanokapsel intakt bleibt,
wobei die duftende Zusammensetzung
- demineralisiertes Wasser,
- einen Viskositätsmodifikator,
- Natriumchlorid und
- ein Konservierungsmittel umfasst,
wobei der Viskositätsmodifikator aus Cellulosegummi oder einem Acrylcopolymer ausgewählt ist, und wobei die Nanokapseln durch die Methode der Nanoverkapselung hergestellt sind und wobei der vorliegende Auffrischer so ausgestaltet ist, dass er die Nanokapseln auf Oberflächen oder Textilien aufsprüht oder aufstreut.

2. Auffrischer für Räume und Gegenstände nach einem der Ansprüche 1 bis 3, **dadurch gekennzeichnet, dass** das Konservierungsmittel 5-Chlor-2-methyl-4-isothiazolin-3-on, 2-Methyl-4-isothiazolin-3-on und Benzylalkohol ist.

3. Auffrischer für Räume und Gegenstände nach einem der Ansprüche 1 bis 3, **dadurch gekennzeichnet, dass** das Konservierungsmittel 1,2-Benzisothiazol-3(2H)-on und 3(2H)-Isothiazolon, 2-Methyl- ist.

4. Auffrischer für Räume und Gegenstände nach einem der Ansprüche 1 bis 3, **dadurch gekennzeichnet, dass** das Konservierungsmittel 1,2-Benzisothiazol-3(2H)-on und 2-Methyl-(2H)-isothiazol-3-on ist.

5. Auffrischer für Räume und Gegenstände nach einem der Ansprüche 1 bis 3, **dadurch gekennzeichnet, dass** das Konservierungsmittel 1,3-Bis(hydroxymethyl)-5,5-dimethylimidazolidin-2,4-dion und Formaldehyd ist.

6. Auffrischer für Räume und Gegenstände nach einem der Ansprüche 1 bis 3, **dadurch gekennzeichnet, dass** das Konservierungsmittel 1,2-Benzisothiazol-3(2H)-on, 2-Methyl-2H-isothiazol-3-on und N-(3-Aminopropyl)-N-dodecylpropan-1,3-diamin ist.

7. Auffrischer für Räume und Gegenstände nach einem der vorstehenden Ansprüche, **dadurch gekennzeichnet, dass** die Nanokapseln solche Eigenschaften aufweisen, dass nach Einwirkung einer Kraft auf die Kapsel die Membran der Duftkapsel aufbricht und der Duft sofort freigesetzt wird.

8. Auffrischer für Räume und Gegenstände nach einem der vorstehenden Ansprüche, **dadurch gekennzeichnet, dass** die Nanokapseln solche Eigenschaften aufweisen, dass nach Einwirkung einer Kraft auf die Kapsel die Membran der Duftkapsel aufspringt und nach Freisetzung der anfänglich spürbaren Duftmenge der Duft über einen bestimmten Zeitraum bis zu mehreren Monaten weiter freigesetzt wird.

9. Auffrischer für Räume und Gegenstände nach einem der Ansprüche 1 bis 6, **dadurch gekennzeichnet, dass** die Kapseln solche Eigenschaften aufweisen, dass nach Aktivierung der Kapsel durch Berührung eine bessere / verstärkte sofortige Duftfreisetzung erfolgt, zugleich jedoch auch eine verlängerte Wirkung / Duftabgabe gegeben ist.

10. Auffrischer für Räume und Gegenstände nach einem der Ansprüche 1 bis 6, **dadurch gekennzeichnet, dass** die Kapseln solche Eigenschaften aufweisen, dass nach Einwirkung einer Kraft auf die Kapsel die Membran der Duftkapsel aufspringt und nach Freisetzung der anfänglich spürbaren Duftmenge der Duft über einen bestimmten Zeitraum bis zu mehreren Monaten weiter freigesetzt wird und / oder nach Einwirkung einer Kraft auf die Kapsel die Membran der Duftkapsel aufbricht und der Duft sofort freigesetzt wird und / oder nach Aktivierung der Kapsel durch Berührung eine bessere / verstärkte sofortige Duftfreisetzung erfolgt, zugleich jedoch auch eine verlängerte Wirkung / Duftabgabe gegeben ist.

11. Auffrischer für Räume und Gegenstände nach einem der vorstehenden Ansprüche, **dadurch gekennzeichnet, dass** er zur Auffrischung von Geweben und Textilien verwendet wird.

## Revendications

1. Désodorisant d'espaces et d'objets comprenant des nano-capsules contenant une composition parfumée,
**caractérisé par le fait que** la composition parfumée est configurée :
- pour libérer le parfum de la composition parfumée dans l'espace lorsqu'une seule nanocapsule se brise, et
- pour garantir la fraîcheur, le parfum étant protégé dans chaque nano-capsule tant qu'elle reste intacte,
la composition parfumée comprenant :
- de l'eau déminéralisée,
- un modificateur de viscosité,
- du chlorure de sodium, et
- un agent de conservation,
le modificateur de viscosité étant sélectionné parmi la gomme de cellulose ou un copolymère acrylique, et les nano-capsules étant produites par une méthode de nano-encapsulation, ledit désodorisant étant conçu pour pulvériser ou saupoudrer les nano-capsules sur des surfaces ou des tissus.

2. Désodorisant d'espaces et d'objets selon l'une quelconque des revendications 1 à 3, **caractérisé en ce que** l'agent de conservation est le 5-chloro-2-méthyl-4-isothiazolin-3-one, le 2-méthyl-4-isothiazolin-3-one et l'alcool benzylique.

3. Désodorisant d'espaces et d'objets selon l'une quelconque des revendications 1 à 3, **caractérisé en ce que** l'agent de conservation est le 1,2-benzisothiazol-3(2H)-one et le 3(2H)-isothiazolone, 2-méthyl-.

4. Désodorisant d'espaces et d'objets selon l'une quelconque des revendications 1 à 3, **caractérisé en ce que** l'agent de conservation est le 1,2-benzisothiazol-3(2H)-one et le 2-méthyl-(2H)-isothiazol-3-one.

5. Désodorisant d'espaces et d'objets selon l'une quelconque des revendications 1 à 3, **caractérisé en ce que** l'agent de conservation est le 1,3-bis(hydroxyméthyl)-5,5-diméthylimidazolidine-2,4-dione et le formaldéhyde.

6. Désodorisant d'espaces et d'objets selon l'une quelconque des revendications 1 à 3, **caractérisé en ce que** l'agent de conservation est le 1,2-benzisothiazol-3(2H)-one, le 2-méthyl-2H-isothiazol-3-one et le N-(3-aminopropyl)-N-dodécylpropane-1,3-diamine.

7. Désodorisant d'espaces et d'objets selon l'une quelconque des revendications précédentes, **caractérisé en ce que** les nano-capsules ont de telles caractéristiques qu'après l'application d'une force sur la capsule, la membrane de la capsule de parfum se brise et le parfum qu'elle contient est libéré instantanément.

8. Désodorisant d'espaces et d'objets selon l'une quelconque des revendications précédentes 1 à 6, **caractérisé en ce que** les capsules ont de telles caractéristiques qu'après l'application d'une force sur la capsule, la membrane de la capsule de parfum se fissure et, après la libération d'une quantité initiale perceptible de parfum, celui-ci continue d'être diffusé pendant une certaine période allant jusqu'à plusieurs mois.

9. Désodorisant d'espaces et d'objets selon l'une quelconque des revendications précédentes 1 à 6, **caractérisé en ce que** les capsules ont de telles caractéristiques qu'après activation de la capsule par contact, celle-ci réagit par une libération instantanée améliorée / renforcée du parfum, mais présente également un effet prolongé de diffusion du parfum.

10. Désodorisant d'espaces et d'objets selon l'une quelconque des revendications précédentes 1 à 6, **caractérisé en ce que** les capsules ont de telles caractéristiques qu'après l'application d'une force sur la capsule, la membrane de la capsule de parfum se fissure et, après la libération d'une quantité initiale perceptible de parfum, celui-ci continue d'être diffusé pendant une certaine période allant jusqu'à plusieurs mois, et / ou qu'après l'application d'une force sur la capsule, la membrane de la capsule de parfum se brise et le parfum est libéré instantanément, et / ou qu'après activation de la capsule par contact, celle-ci réagit par une libération instantanée améliorée / renforcée du parfum, mais présente également un effet prolongé de diffusion du parfum.

11. Désodorisant d'espaces et d'objets selon l'une quelconque des revendications précédentes, **caractérisé en ce qu'**il est utilisé pour le rafraîchissement des tissus et textiles.
